(19) Europäisches Patentamt / European Patent Office / Office européen des brevets

(11) **EP 4 467 095 A1**

(12) **EUROPEAN PATENT APPLICATION**
published in accordance with Art. 153(4) EPC

(43) Date of publication:
27.11.2024 Bulletin 2024/48

(21) Application number: 23743116.8

(22) Date of filing: 06.01.2023

(51) International Patent Classification (IPC):
*A61B 34/10* (2016.01)   *A61B 34/20* (2016.01)
*A61B 34/37* (2016.01)   *H04N 13/302* (2018.01)
*H04N 13/361* (2018.01)

(52) Cooperative Patent Classification (CPC):
A61B 34/10; A61B 34/20; A61B 34/37;
H04N 13/302; H04N 13/361

(86) International application number:
PCT/JP2023/000118

(87) International publication number:
WO 2023/140120 (27.07.2023 Gazette 2023/30)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 21.01.2022 JP 2022007631

(71) Applicant: Sony Group Corporation
Tokyo 108-0075 (JP)

(72) Inventors:
• KATSUKI Shinji
  Tokyo 108-0075 (JP)
• MATSUURA Kana
  Tokyo 108-0075 (JP)
• KOBAYASHI Motoaki
  Tokyo 108-0075 (JP)
• MATSUURA Hiromitsu
  Tokyo 108-0075 (JP)

(74) Representative: D Young & Co LLP
3 Noble Street
London EC2V 7BQ (GB)

(54) **SURGICAL ROBOT SYSTEM**

(57)    The present technology relates to a surgical robot system that makes it easy for a medical staff such as an operator to perceive a structure of a living body by 3D display of a surgical video in surgery using a surgical robot, a surgical microscope, or the like, and enables efficient surgery.

Included are: a spatial reproduction display capable of displaying a surgical video visually recognized as a stereoscopic image in a stereoscopic space formed by a plane through which a lower side of a display surface of a monitor and a normal surface of an installation surface pass and a plane through which an upper side of the display surface and a parallel surface of the installation surface pass; an imaging device that captures the surgical video; an operating device connected to the display; and a surgical robot that operates by an operation of an operator via the operating device. The present technology can be applied to a surgical robot system.

*FIG. 12*

EP 4 467 095 A1

## Description

TECHNICAL FIELD

The present technology relates to a surgical robot system, and for example, relates to a surgical robot system configured to provide a video that allow a medical staff to perform surgery efficiently.

BACKGROUND ART

In surgery using a surgical robot, a surgical microscope, or the like, a surgical video is displayed in 3D so that a medical staff such as a surgeon can easily perceive a structure of a living body and can perform surgery efficiently (Patent Document 1).

CITATION LIST

PATENT DOCUMENT

Patent Document 1: Japanese Patent Application Laid-Open No. 2019-97890

SUMMARY OF THE INVENTION

PROBLEMS TO BE SOLVED BY THE INVENTION

Provided is a video image that allows a medical staff to perform surgery more efficiently.
The present technology has been made in view of such a situation, and an object thereof is to provide a video image that allows a medical staff to perform surgery more efficiently.

SOLUTIONS TO PROBLEMS

A surgical robot system according to one aspect of the present technology is a surgical robot system including: a spatial reproduction display capable of displaying a surgical video visually recognized as a stereoscopic image in a stereoscopic space formed by a plane through which a lower side of a display surface of a monitor and a normal surface of an installation surface pass and a plane through which an upper side of the display surface and a parallel surface of the installation surface pass; an imaging device that captures the surgical video; an operating device connected to the spatial reproduction display; and a surgical robot that operates by an operation of an operator via the operating device.
A surgical robot system according to one aspect of the present technology includes: a spatial reproduction display capable of displaying a surgical video visually recognized as a stereoscopic image in a stereoscopic space formed by a plane through which a lower side of a display surface of a monitor and a normal surface of an installation surface pass and a plane through which an upper side of the display surface and a parallel surface of the installation surface pass; an imaging device that captures the surgical video; an operating device connected to the spatial reproduction display; and a surgical robot that operates by an operation of an operator via the operating device.

BRIEF DESCRIPTION OF DRAWINGS

Fig. 1 is a diagram illustrating a configuration in an embodiment of a surgical robot system to which the present technology is applied.
Fig. 2 is a diagram illustrating an example of stereoscopic display by a stereoscopic display.
Fig. 3 is a diagram illustrating an example of stereoscopic display by the stereoscopic display.
Fig. 4 is a diagram illustrating an example of stereoscopic display by the stereoscopic display.
Fig. 5 is a diagram for explaining stereoscopic display to which the present technology is applied.
Fig. 6 is a diagram illustrating a configuration of an embodiment of an information processing device.
Fig. 7 is a diagram illustrating an installation example of a display unit.
Fig. 8 is a diagram for explaining a stereoscopic effect perceived by a user.
Fig. 9 is a flowchart illustrating an operation example of the present embodiment.
Fig. 10 is a diagram for explaining display control processing by a display control unit.
Fig. 11 is a diagram for explaining the display control processing by the display control unit.
Fig. 12 is a diagram illustrating an example of a configuration of a surgical robot system.
Fig. 13 is a diagram for explaining a surgical video displayed on a spatial reproduction display.
Fig. 14 is a diagram illustrating an example of a composite image.
Fig. 15 is a diagram illustrating an example of a space CG.
Fig. 16 is a diagram illustrating a difference in a composite image depending on a viewpoint position.
Fig. 17 is a diagram illustrating an example of a composite image.
Fig. 18 is a diagram illustrating an example of a composite image.
Fig. 19 is a diagram illustrating an example of a composite image.
Fig. 20 is a diagram illustrating an example of a composite image.
Fig. 21 is a diagram illustrating an example of a composite image.
Fig. 22 is a diagram illustrating an example of a composite image.
Fig. 23 is a diagram illustrating an example of a composite image.
Fig. 24 is a diagram illustrating an example of a composite image.
Fig. 25 is a diagram illustrating an example of a composite image.
Fig. 26 is a diagram illustrating an example of a composite image.
Fig. 27 is a diagram illustrating an example of a composite image.
Fig. 28 is a diagram illustrating an example of another configuration of the surgical robot system.
Fig. 29 is a diagram illustrating an example of a composite image.
Fig. 30 is a diagram illustrating an example of a composite image.

MODE FOR CARRYING OUT THE INVENTION

The following is a description of modes (hereinafter referred to as embodiments) for carrying out the present technology.

1. Surgical robot system
2. Spatial reproduction display
3. Surgical video realized by spatial reproduction display

<1. Surgical robot system>

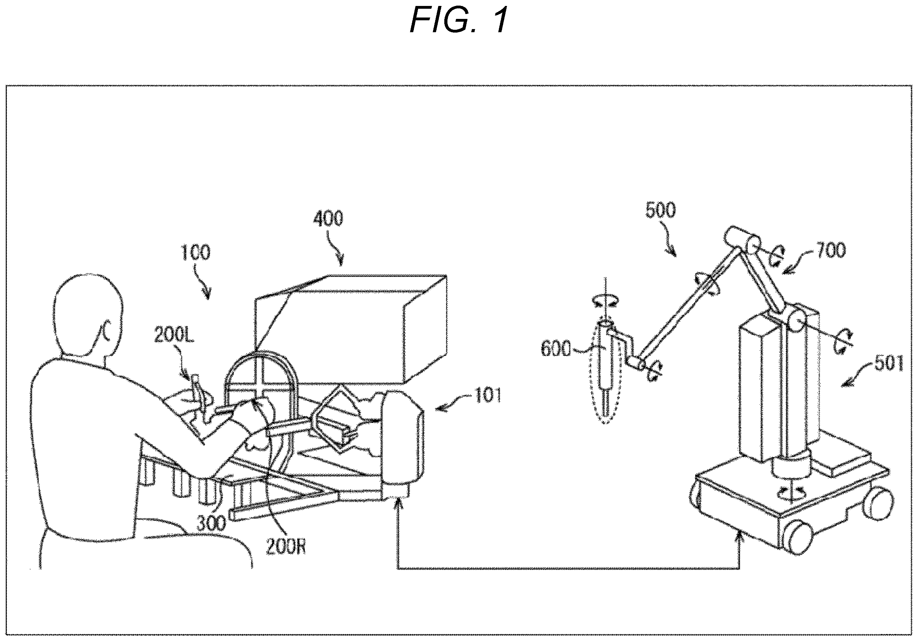

Fig. 1 is a diagram illustrating a configuration of a surgical robot system according to an embodiment of the present technology.

The surgical robot system includes a master device 100 and a slave device 500. In the surgical robot system, a user drives the slave device 500 by controlling the master device 100 to perform surgery.

The master device 100 is an information processing device having an input function for operating the slave device 500, a control function of the slave device 500, and a presentation function of presenting a signal output from a camera or a sensor of the slave device 500 to the user.

As illustrated in Fig. 1, the master device 100 includes an information processing device 101 (first information processing device), an input device 200 (right hand input device 200R and left hand input device 200L) gripped and operated by the user, a support base 300 on which both arms, both elbows, or the like of the user are placed, and a display device 400 that displays a video on the basis of a signal from the slave device 500.

The master device 100 controls the slave device 500 by the information processing device 101 outputting a control signal to the slave device 500 on the basis of an input of the input device 200. In the master device 100, the information processing device 101 performs vibration (tactile presentation) to the input device 200 or display to the display device 400 on the basis of the signal from the slave device 500, thereby presenting the video of the operative field and feedback from the slave device 500 to the user.

The information processing device 101 is a device that processes information in the master device 100, and is, for example, a computer having an information processing circuit including a CPU, a memory, and the like.

The input device 200 is a device that receives a user's input, and is, for example, a device that can input a hand operation or a gripping operation of the user.

The support base 300 is a base on which both arms or both elbows of the user can be placed, and is, for example, an aluminum frame. Note that a device such as a touch display capable of inputting to the master device 100 and the slave device 500 may be further provided on the support base 300.

The display device 400 is a display device capable of displaying a virtual three-dimensional object on a predetermined space so that the user can visually recognize the virtual three-dimensional object, and is, for example, a spatial reproduction display. In the display device 400, the display surface is obliquely disposed on a far side with respect to the user.

Note that a degree of inclination of the display surface may be changeable on the basis of an arrangement angle of the input device 200. In the example of Fig. 1, the display device 400 is disposed on a back side of the input device 200, but may be disposed on a front side of the input device 200. At this time, a CG image that changes in accordance with the user's input is preferably included in an image displayed by the display device 400. As a result, the user can obtain an illusion as if the user directly touches the object in the space virtually reproduced by the display device 400.

The slave device 500 includes an information processing device 501 (second information processing device), a medical device 600, and an arm 700 that supports the medical device.

In the slave device 500, the information processing device 501 controls the medical device 600 or the arm 700 on the basis of the signal transmitted from the master device 100, thereby performing control based on the user's input.

The information processing device 501 is a device that processes information in the slave device 500, and is, for example, a computer having an information processing circuit including a CPU, a memory, and the like.

The medical device 600 is a device that operates on the basis of an input of the master device 100, and is, for example, an imaging device such as a 3D imaging device or an endoscope, or an end effector including an electric scalpel or forceps.

The slave device 500 preferably includes a plurality of sets of the medical device 600 and the arm 700. For example, a first arm including an imaging device and a second arm including an end effector may be provided.

The end effector preferably includes a detection device that detects contact with an affected area or the like. As a result, it is possible to perform appropriate feedback to the user by vibrating the input device or restricting the operation on the basis of the pressure on the end effector.

<2. Spatial reproduction display>

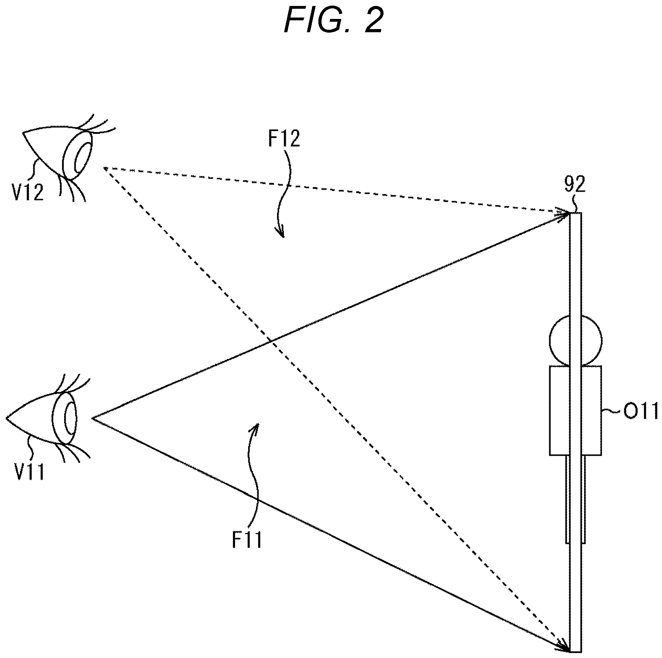

Next, a spatial reproduction display that is a stereoscopic display applied as a display device of the surgical robot system will be described with reference to Figs. 2 to 9.

In recent years, a naked-eye stereoscopic image display device (Hereinafter, also referred to as a naked eye stereoscopic display or simply a stereoscopic display.) capable of stereoscopically displaying content without using dedicated glasses has been proposed. Such a naked-eye stereoscopic display can display an image shifted in the horizontal direction for each viewing angle, and when the user views different images with the left eye and the right eye, the content can be observed stereoscopically.

In such a stereoscopic display, in order to enhance the stereoscopic effect felt by the user and the feeling as if the content exists in the same space (Hereinafter, also referred to as a feeling of coexistence), for example, it is desirable to increase a parallax amount caused by the displacement of the right-eye image and the left-eye image in the horizontal direction.

Figs. 2 to 4 are explanatory diagrams illustrating an example of stereoscopic display by the stereoscopic display. In the example illustrated in Fig. 2, a stereoscopic object O11 (an example of content) is displayed in the vicinity of a display surface 92 of the stereoscopic display.

In the example illustrated in Fig. 2, the user can observe from a relatively free viewpoint position, and for example, in a case where the user observes at a viewpoint V11, an object O11 is included in a field of view F11, and in a case where the user observes at a viewpoint V12, an object O11 is included in a field of view F12. However, since the user perceives the depth of the object O11 with reference to the display surface 92, it is difficult to obtain a sufficient stereoscopic effect in the example of Fig. 2 in which the object O11 is displayed in the vicinity of the display surface 92. In the example illustrated in Fig. 2, since the entire object O11 is present in the vicinity of the display surface 92, the sense of being displayed on the stereoscopic display is strong, and it is also difficult to obtain a sufficient feeling of coexistence.

In order to enhance the stereoscopic effect and the coexistence feeling, for example, it is conceivable to dispose the object so as to be perceived on a front side or a back side of the display surface 92.

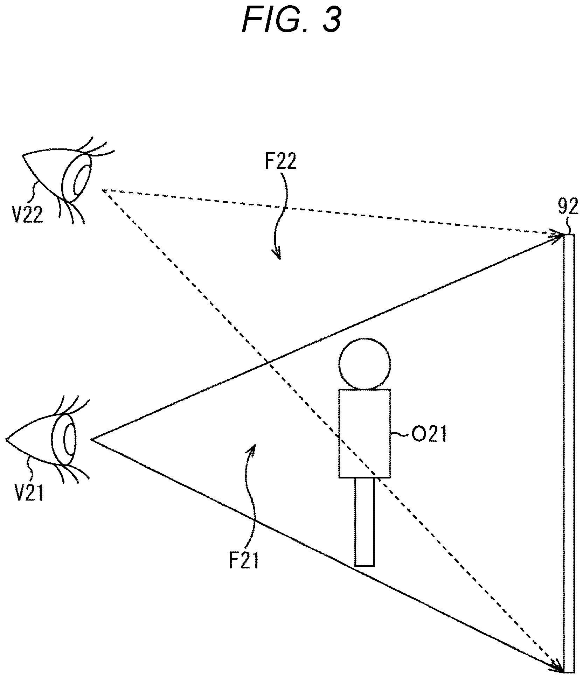

In the example illustrated in Fig. 3, an object O21 is disposed on the front side of the display surface 92 of the stereoscopic display, and in the example illustrated in Fig. 3, an object O31 is disposed on the back side of the display surface 92 of the stereoscopic display.

However, when the object is disposed on the front side of the display surface 92 as illustrated in Fig. 3, the object is displayed larger than the example illustrated in Fig. 2, and thus, there is a case where the viewpoint position at which the user can three-dimensionally observe the object O21 is limited as compared with the example illustrated in Fig. 2. In the example illustrated in Fig. 3, in a case where the user observes at a viewpoint V21, the object O21 is completely included in a field of view F21, but in a case where the user observes at a viewpoint V22, some of the objects O21 are not included in a field of view F22. Therefore, in a case where the user observes the object O21 from the viewpoint V22, a so-called sticking effect (frame effect) occurs, and it may be difficult to stereoscopically observe the object O21.

In a case where the object is disposed on the front side of the display surface 92 as illustrated in Fig. 3, the parallax amount (binocular parallax amount) perceived by the user is larger than that in the example illustrated in Fig. 2. When content is observed with a large parallax amount, it becomes difficult to fuse images, and fatigue and motion sickness are likely to occur, which may increase a burden on the user.

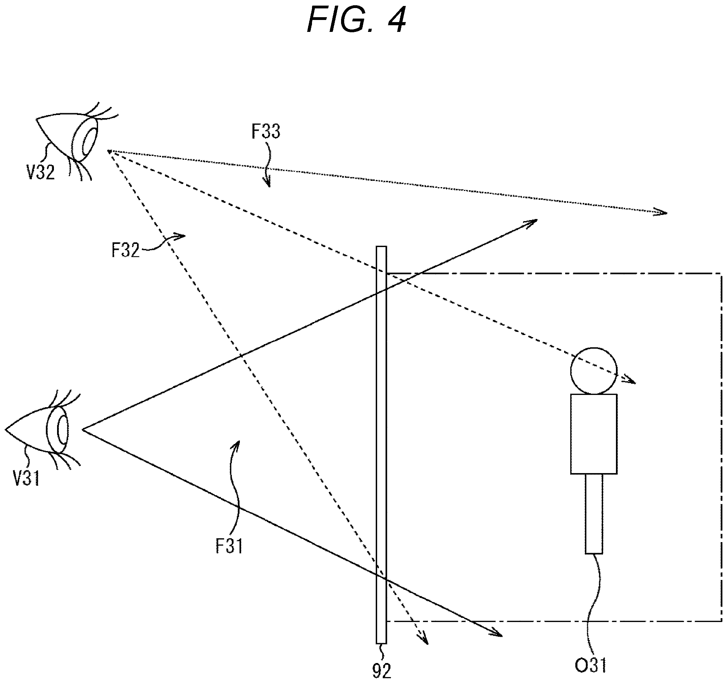

As illustrated in Fig. 4, when an object is disposed on the back side of the display surface 92, the user perceives that the object O31 exists in a box existing on the back side of the display surface 92. Therefore, the field of view of the user is limited (narrowed), and the viewpoint position at which the user can stereoscopically observe the object O31 may be limited as compared with the example illustrated in Fig. 2.

In the example illustrated in Fig. 4, the object O31 is completely included in a field of view F31 in a case where the user observes from a viewpoint V31, but a part of the object O31 is not included in a field of view F32 in a case where the user observes from a viewpoint V32. Here, since the object O31 is displayed on the back side of the display surface 92 even though the field of view of the user is a range obtained by combining the field of view F32 and a field of view F33, the field of view for the user to observe the content is limited to the field of view F32.

Even in a case where an object is disposed on the back side of the display surface 92 as illustrated in Fig. 4, the amount of parallax perceived by the user increases similarly to the example illustrated in Fig. 3, and thus, there has been a possibility that a burden on the user increases.

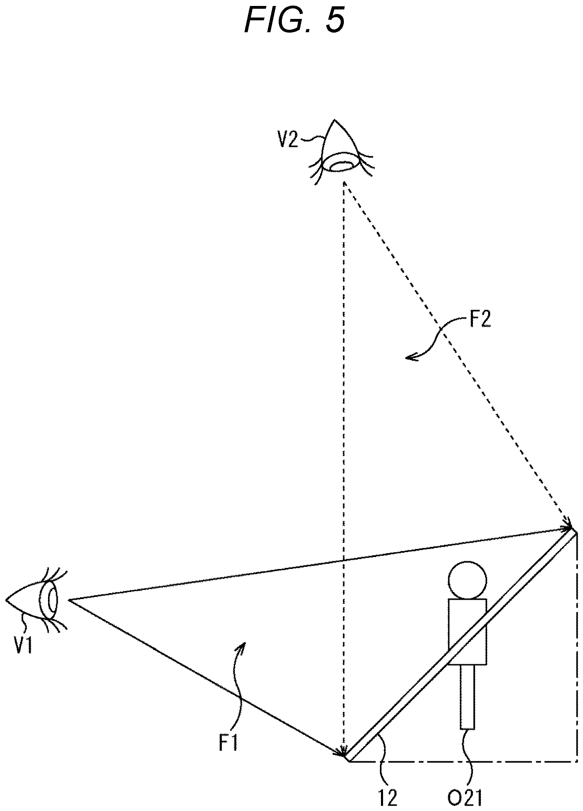

From the above, one embodiment of the present disclosure has been created. By using the stereoscopic display (spatial reproduction display) of the present embodiment in the surgical robot system, it is possible for the user to observe the stereoscopic image in a wider range while suppressing the burden on the user to observe the stereoscopic image. Fig. 5 is an explanatory diagram for describing an outline of the present embodiment.

In the examples illustrated in Figs. 2 to 4, the stereoscopic display is installed such that the display surface 92 of the stereoscopic display is perpendicular to the horizontal plane in the real space. On the other hand, in the example illustrated in Fig. 5, the stereoscopic display is installed such that a display surface 12 of the stereoscopic display is inclined (non-perpendicular) with respect to the horizontal plane in the real space. In the example illustrated in Fig. 5, an object O1 (stereoscopic object) is disposed so as to cross the display surface 12 while standing upright with respect to the horizontal plane.

With such a configuration, it is possible to give a feeling of coexistence such that the object O1 exists in the same space as the user without causing the user to feel the display surface. By disposing the floor surface and the wall surface as described later while suppressing the parallax amount as compared with the examples illustrated in Figs. 3 and 4, it is possible to give a sufficient stereoscopic effect and to suppress the burden on the user.

As illustrated in Fig. 5, the object O1 is included in a field of view F1 in a case where the user observes from a viewpoint V1, and the object O1 is included in a field of view F2 in a case where the user observes from a viewpoint V2. Therefore, the user can observe from a wider viewpoint position.

With such a configuration, by providing the user with an operative field video virtually reproduced so as to exist in the space, the medical staff can perform the surgery more efficiently.

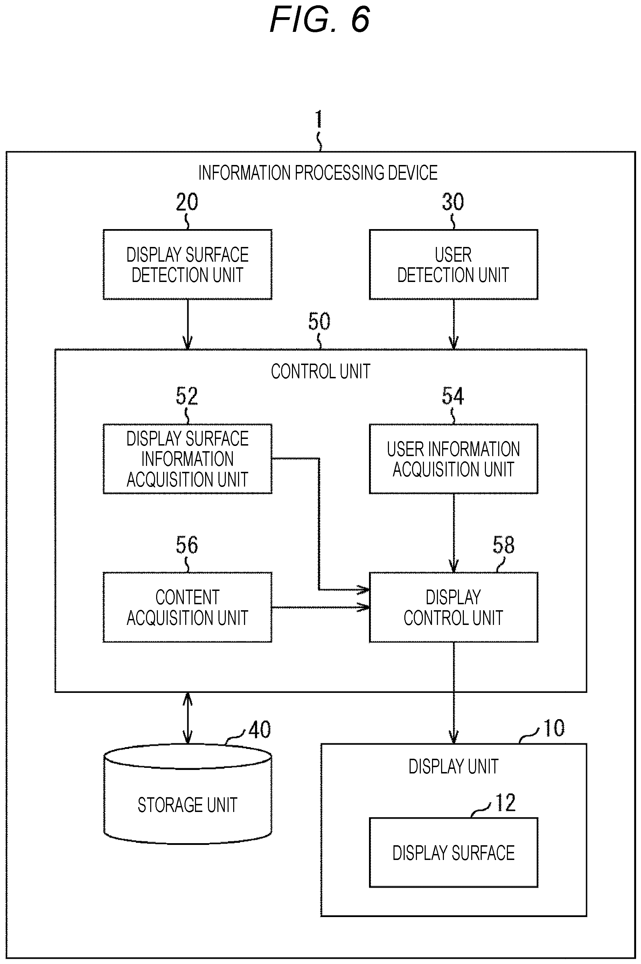

The overview of one embodiment of the present disclosure has been described above. Next, a configuration according to the present embodiment will be described. Fig. 6 is a block diagram illustrating a configuration example of an information processing device 1 according to the present embodiment.

As illustrated in Fig. 6, the information processing device 1 according to the present embodiment includes a display unit 10, a display surface detection unit 20, a user detection unit 30, a storage unit 40, and a control unit 50.

The display unit 10 is a display that displays a stereoscopic image under the control of the control unit 50 as described later. For example, the display unit 10 may be a naked-eye stereoscopic display (naked-eye stereoscopic image display device). In the display unit 10, a surface on which a stereoscopic image is displayed is the display surface 12.

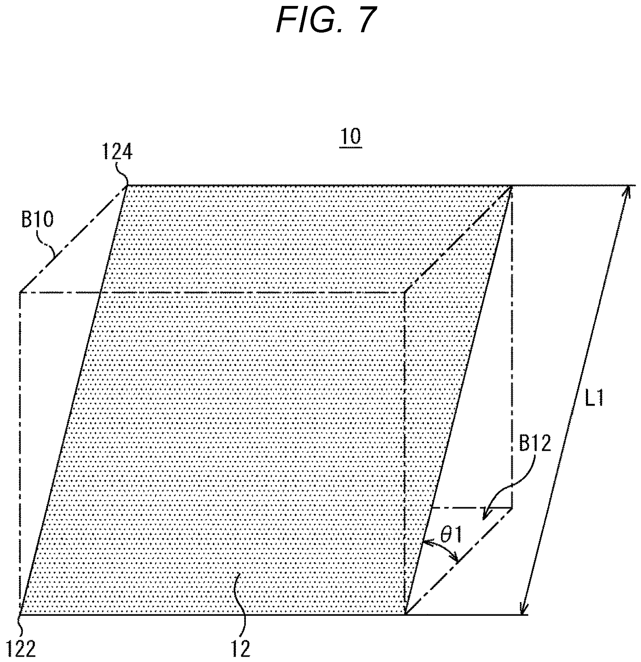

As described with reference to Fig. 5, the display unit 10 according to the present embodiment can be installed such that the display surface 12 is oblique (non-perpendicular) to the horizontal plane in the real space. Fig. 7 is an explanatory diagram illustrating an installation example of the display unit 10 according to the present embodiment. Note that, although not illustrated in Fig. 7, the display unit 10 may have a mechanism (housing) that supports the display surface 12.

As illustrated in Fig. 7, a distance from a lower end 122 of the display surface 12 to an upper end 124 of the display surface 12 is defined as L1. A space having the lower end 122 of the display surface 12 and the upper end 124 of the display surface 12 as diagonal sides is set as a drawing space B10 by the control unit 50 as described later. Here, a bottom surface B12 of the drawing space B10 is a surface horizontal to the real space (horizontal plane in the real space). Note that the bottom surface B12 is desirably substantially the same surface as the installation surface of the display unit 10.

As illustrated in Fig. 7, the display unit 10 is disposed such that the horizontal plane (bottom surface B12) of the real space and the display surface 12 have an angle θ1 larger than 0° and smaller than 90°. The arrangement method is not particularly limited, and the arrangement may be realized by the shape of the display unit 10, or the arrangement may be realized by supporting the display unit 10 by an instrument (stand or the like) different from the display unit 10.

The display surface detection unit 20 illustrated in Fig. 6 detects an orientation of the display surface 12 and provides the orientation to the control unit 50. Here, the orientation of the display surface 12 detected by the display surface detection unit 20 may be, for example, an angle (angle θ1 illustrated in Fig. 6) formed by the horizontal plane and the display surface 12 in the real space. The display surface detection unit 20 may be realized by, for example, any one of an acceleration sensor, a gyro sensor, and a magnetic sensor having a predetermined relationship with the display surface 12, or a combination of the above.

The user detection unit 30 detects information regarding the user observing the stereoscopic image and provides the information to the control unit 50. The user detection unit 30 may detect a position and a posture of the user, and the position and the posture of the user may be, for example, a relative position and posture with respect to the display surface 12. The position of the user may be the position of the user's eye, or may be the position of the user's left eye and the position of the user's right eye. The posture of the user may be a direction of the face of the user or a line-of-sight direction of the left eye and the right eye of the user.

The user detection unit 30 may be realized by, for example, any one of a camera, a depth camera, and a human sensor, or a combination of the above.

The storage unit 40 stores programs and parameters for each configuration of the information processing device 1 to function. The storage unit 40 may store content data. The data of the content stored in the storage unit 40 may include, for example, data of a stereoscopic object (3D image), a planar object (2D image), audio, or the like. The storage unit 40 may include information regarding the display surface, and the information regarding the display surface may include, for example, information on the distance (distance L1 illustrated in Fig. 7) from the lower end to the upper end of the display surface.

The control unit 50 controls each configuration of the information processing device 1. As illustrated in Fig. 5, the control unit 50 also functions as a display surface information acquisition unit 52, a user information acquisition unit 54, a content acquisition unit 56, and a display control unit 58.

The display surface information acquisition unit 52 acquires display surface information related to the display surface 12. For example, the display surface information acquisition unit 52 may acquire, as the display surface information, information of an angle (posture of the display surface 12) formed by a horizontal plane and the display surface 12 in the real space from the display surface detection unit 20. The display surface information acquisition unit 52 may acquire information on the distance from the lower end to the upper end of the display surface from the storage unit 40 as the display surface information. The display surface information acquisition unit 52 provides the acquired display surface information to the display control unit 58.

The user information acquisition unit 54 acquires user information regarding the user. For example, the user information acquisition unit 54 acquires, as the user information, information of the position (position of left eye of user and position of right eye of user) and the posture of the user from the user detection unit 30.

Note that the user information acquisition unit 54 may directly or indirectly acquire the information on the position and posture of the user from the user detection unit 30. For example, in a case where the user detection unit 30 is a camera oriented in an observation direction of the display surface 12, the user information acquisition unit 54 may specify and indirectly acquire the user information on the basis of the image provided from the user detection unit 30. The user information acquisition unit 54 provides the acquired user information to the display control unit 58.

The content acquisition unit 56 acquires data of content related to display. For example, the content acquisition unit 56 reads and acquires content data stored in the storage unit 40. The content acquisition unit 56 provides data of the acquired content to the display control unit 58.

The display control unit 58 controls display on the display unit 10 on the basis of the display surface information provided from the display surface information acquisition unit 52, the user information provided from the user information acquisition unit 54, and the data of the content provided from the content acquisition unit 56.

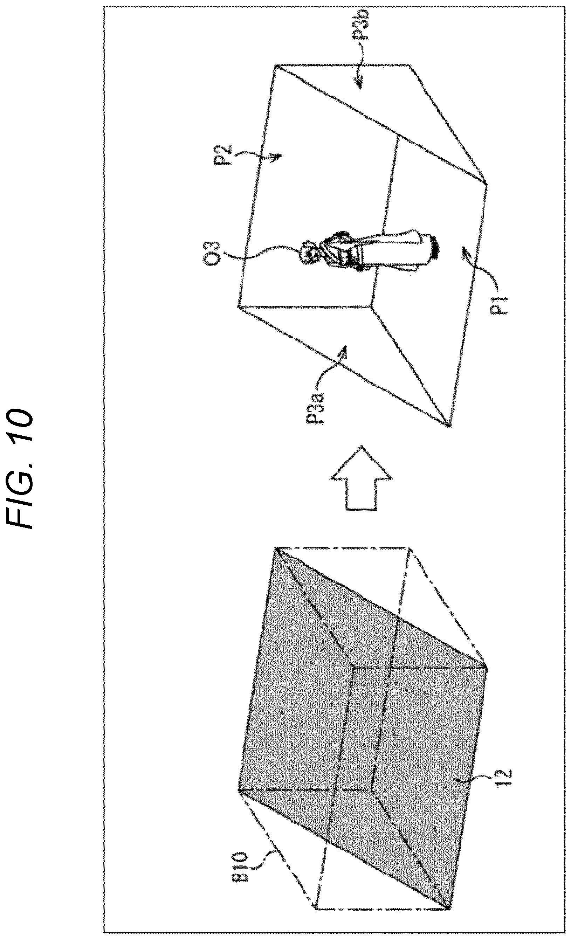

The display control unit 58 may render the drawing space described with reference to Fig. 7 according to the position of the user (positions of left eye and right eye), generate a stereoscopic image (left-eye image and right-eye image) by performing orthographic projection according to the position of the user, and display the stereoscopic image on the display surface 12 of the display unit 10. With such a configuration, the display control unit 58 does not need to generate images of all viewpoints (viewing angles) that can be displayed by the display unit 10, and is only required to generate images only for two viewpoints, so that the processing amount can be suppressed. Note that details of the generation of the stereoscopic image according to the position of the user will be described later with reference to Fig. 10.

For example, the display control unit 58 may display the stereoscopic image on the display surface 12 of the display unit 10 such that a floor surface (first plane) parallel to the horizontal plane is observed in a region according to a distance from the lower end to the upper end of the display surface 12 and an angle formed by the horizontal plane and the display surface 12 in the real space. The display control unit 58 may display the stereoscopic image on the display surface 12 of the display unit 10 such that a back wall surface (second plane) that is in contact with the upper end of the display surface and is perpendicular to the floor surface described above (that is, perpendicular to the horizontal plane) is observed. The display control unit 58 may display the stereoscopic image on the display surface 12 of the display unit 10 such that the stereoscopic object disposed on the floor surface is observed.

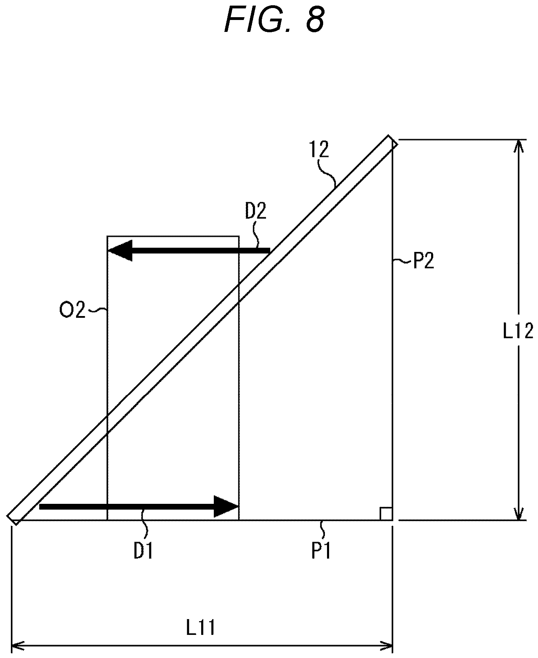

Fig. 8 is an explanatory diagram for explaining a stereoscopic effect perceived by the user by the stereoscopic image displayed by the display control unit 58.

As illustrated in Fig. 8, a floor surface P1 parallel to the horizontal plane is observed. As illustrated in Fig. 8, the floor surface P1 is desirably disposed so as to be in contact with a lower end of the display surface 12. As illustrated in Fig. 8, a back wall surface P2 perpendicular to the floor surface P1 is desirably disposed so as to be in contact with an upper end of the display surface 12. With this configuration, drawing can be performed such that the binocular parallax and the motion parallax of the images displayed at the lower end and the upper end of the display surface 12 are reduced (for example, 0).

As described above, since the display control unit 58 generates the stereoscopic image according to the position of the user, there is a possibility that a detection error or a latency (delay) of the position of the user occurs. However, by drawing such that binocular parallax and motion parallax are small at the lower end and the upper end of the display surface 12, even in a case where a detection error or latency occurs, fluctuation is less likely to occur in the vicinity of the lower end and the upper end of the display surface 12, and the user is less likely to feel that the position of the entire content moves (deviates).

In a case where the floor surface P1 and the back wall surface P2 are disposed as described above, a length L11 of the floor surface P1 in the depth direction and a height L12 of the back wall surface P2 can be expressed by the following Formulas (1) and (2) using the distance L1 and the angle $\theta 1$ illustrated in Fig. 7.

$$L11 = L1 \times \cos\theta 1 \qquad (1)$$

$$L21 = L1 \times \sin\theta 1 \qquad (2)$$

With this configuration, the region where the floor surface P1 and the back wall surface P2 are observed is a region according to the actual physical length and arrangement of the display surface 12, and thus, the user can easily recognize (fuse) the floor surface and the back wall surface. As a result, the user can easily recognize the space (drawing space) in which the drawing is performed, and the burden on the stereoscopic observation is suppressed.

As illustrated in Fig. 8, a stereoscopic object O2 disposed on the floor surface P1 is desirably disposed so as to intersect with the display surface 12. With this configuration, the user can perceive parallax according to a distance D1 in the depth direction (retraction direction) and a distance D2 in the pop-out direction (front direction) as illustrated in Fig. 8, and obtain stereoscopic effects in both the depth direction and the pop-out direction. As a result, it is possible to obtain a sufficient stereoscopic effect with a smaller parallax amount as compared with the examples illustrated in Figs. 2 and 3.

Moreover, a microoptical lens is provided on the display surface constituting the display surface 12, and it is preferable that a 4K video signal converted by XGL is divided by the microoptical lens to generate a video for the right eye and a video for the left eye, and the videos are provided to the user.

The display is preferably a display panel of an active retarder method. By adopting the active retarder method, it is possible to visually recognize a 3D video with higher image quality in a case where the user wears circularly polarized glasses. The display panel of the active retarder method is, for example, a display panel in which a liquid crystal cell, a polarizer, and an active retarder in which $\pm \lambda/4$ is alternately switched are stacked, and is a display in which the active retarder alternately switches $\lambda/4$ in accordance with the display of the liquid crystal cell, so that the user can visually recognize a 3D video through polarized glasses.

The user can perceive the position and shape of the stereoscopic object O2 based on the floor surface P1 and the back wall surface P2 recognized as described above, and can obtain a higher coexistence feeling.

The content displayed by the display control unit 58 is not limited to the example described above. For example, the display control unit 58 may display the stereoscopic image on the display surface 12 of the display unit 10 such that a side wall surface (third plane) that is in contact with a left end or a right end of the display surface and is perpendicular to the floor surface (that is, perpendicular to the horizontal plane) is observed. With this configuration, drawing can be performed such that the binocular parallax and the motion parallax of the images displayed at the left end and the right end of the display surface 12 are reduced (for example, 0).

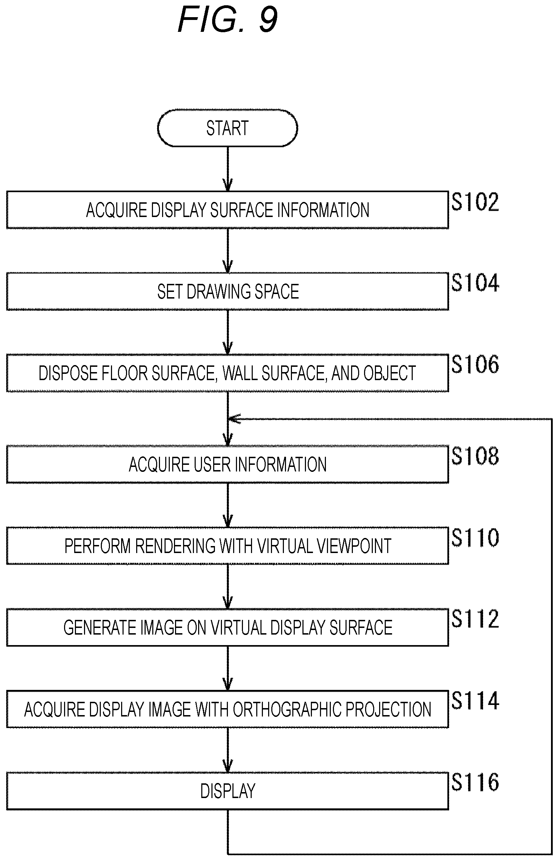

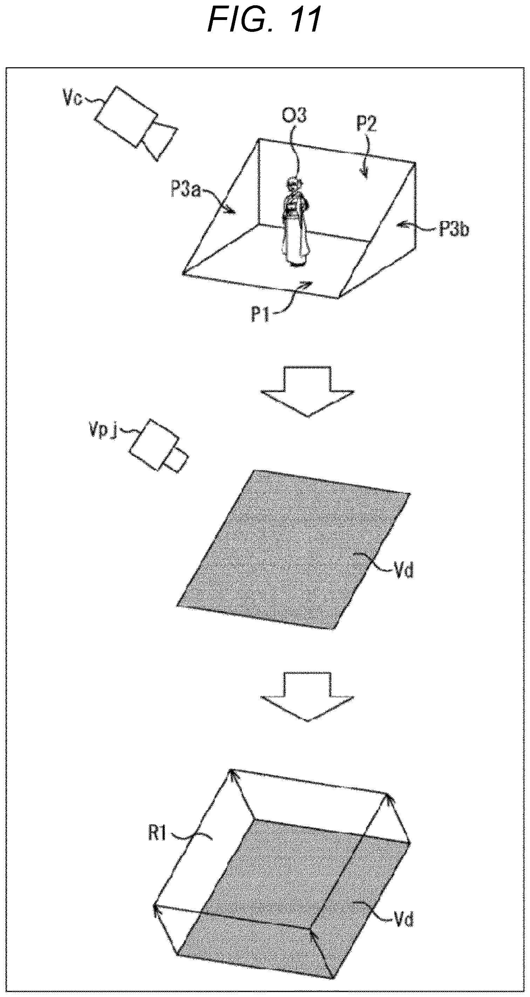

Next, an operation example of the present embodiment will be described with reference to Figs. 9 to 11. Fig. 9 is a flowchart illustrating an operation example of the present embodiment. Figs. 10 and 11 are explanatory diagrams for explaining display control processing by the display control unit 58 of the present embodiment.

As illustrated in Fig. 10, first, the display surface information acquisition unit 52 acquires display surface information (an angle formed by the horizontal plane and the display surface 12 in the real space, and a distance from the lower end to the upper end of the display surface) related to the display surface 12 (S102).

Subsequently, the display control unit 58 sets a drawing space on the basis of the display surface information (S104). For example, as illustrated in Fig. 10, the display control unit 58 may set a drawing space B10 having the upper end and the lower end of the display surface 12 as diagonal sides.

Subsequently, the display control unit 58 disposes the floor surface, the wall surface (back wall surface and side wall surface), and the stereoscopic object in the drawing space (S106). For example, as illustrated in Fig. 10, the display control unit 58 disposes a floor surface P1, a back wall surface P2, a side wall surface P3 (P3a, P3b), and a stereoscopic object O3 on the back side of the display surface 12 in the drawing space.

Subsequently, the user information acquisition unit 54 acquires positions of the left eye and the right eye of the user and a posture of the user as the user information (S108).

As illustrated in Fig. 11, the display control unit 58 virtually installs a virtual camera Vc with the position of the user's eye as a virtual viewpoint, performs rendering of the drawing space described with reference to Fig. 10, and acquires a viewpoint image at the virtual viewpoint (S110).

The display control unit 58 may set a direction of the virtual camera on the basis of the posture of the user (facial orientation or line-of-sight direction), or may set the direction of the virtual camera so that the virtual camera faces the display surface 12.

Subsequently, the display control unit 58 virtually disposes a virtual projector Vpj at the same position and direction as those of the virtual camera Vc, and projects the viewpoint image acquired in step S110 from the virtual projector Vpj onto a virtual display surface Vd virtually installed on the display surface 12 (S112). Here, the virtual projector Vpj may project the viewpoint image with the same parameters (for example, focal length parameters, distortion parameters, and the like) as those of the virtual camera Vc.

Subsequently, the image projected and generated on the virtual display surface Vd in step S112 is orthogonally projected to acquire a display image R1 (S114). The display image R1 becomes a distorted image when viewed from a position other than the current viewpoint position (the position of the user's eye), but is perceived as a normal image when viewed from the current viewpoint position.

Note that, although Fig. 11 illustrates processing for only one viewpoint, the display control unit 58 performs the processing described above at viewpoints for two viewpoints of the left eye and the right eye, and acquires a display image for the left eye and a display image for the right eye. Then, the display control unit 58 causes the display surface 12 to display a combination of the display image for the left eye and the display image for the right eye so that the combination is perceived by the user as a stereoscopic image (S116).

The processing described above in steps S108 to S116 may be repeatedly performed as illustrated in Fig. 9.

Note that the flowchart illustrated in Fig. 9 is an example, and the operation of the present embodiment is not limited to such an example. For example, the entire processing of steps S102 to S116 may be repeatedly performed, and in such a case, the display control unit 58 can perform display control according to a change in the angle between the horizontal plane and the display surface 12.

<3. Surgical video realized by spatial reproduction display>

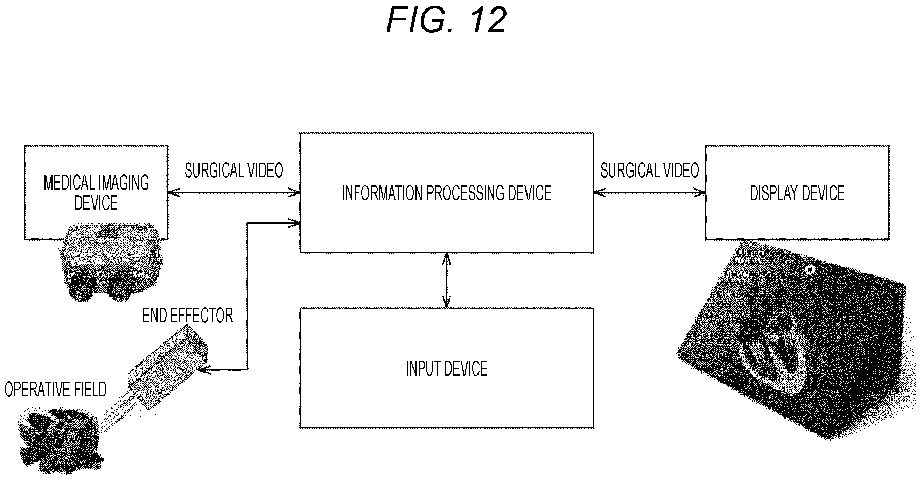

Next, surgical videos realized by the spatial reproduction display will be described with reference to Figs. 12 to 30.

Fig. 12 is a diagram illustrating an example of a configuration of a surgical robot system. Fig. 12 is a diagram illustrating an example of a configuration of the surgical robot system according to the present embodiment. As illustrated in Fig. 12, the surgical robot system of the present embodiment includes an information processing device, a display device, and an input device included in a master device, and a medical imaging device and an end effector included in a slave device.

The display device is a spatial reproduction display, and is a spatial reproduction display that stereoscopically displays a surgical video obtained by imaging the operative field by the medical imaging device on a virtual space. Note that the information processing device preferably includes an FPGA for driving a spatial reproduction display which is a display device. The information processing device may further include an information processing device for controlling the display device different from the information processing device (first information processing device) for controlling the master device, or may be an information processing device integrated with the display device.

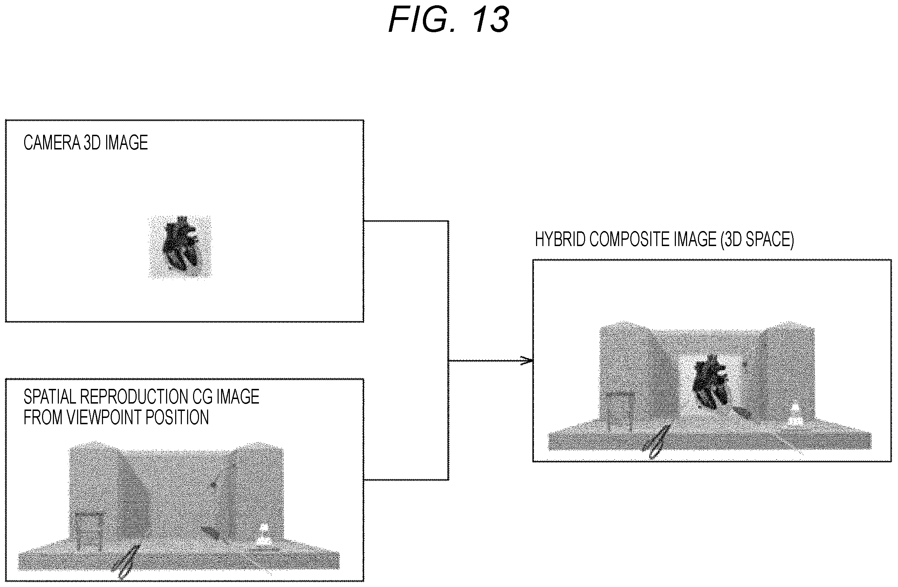

A surgical video realized by the spatial reproduction display will be described. Fig. 13 is a diagram illustrating an example of a composite image. As illustrated in Fig. 13, the information processing device hybridizes a camera 3D image acquired from the medical imaging device and a spatial reproduction CG image from a viewpoint position stored in advance to generate a hybrid composite image (Hereinafter, the composite image) on a 3D space. As a result, it is possible to provide the user with a video in which the user feels that the space is reproduced more.

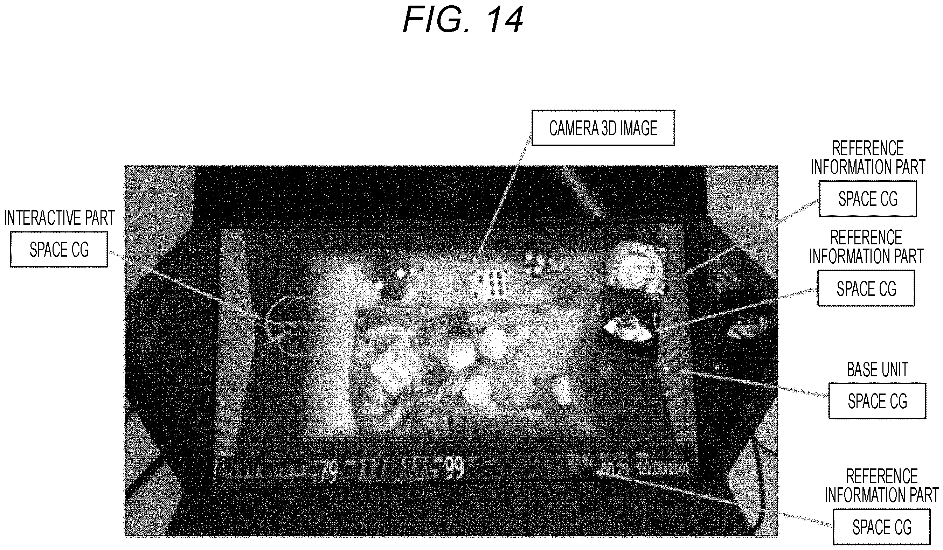

Fig. 14 is a diagram illustrating an example of a composite image. As illustrated in Fig. 14, the information processing device causes the display device to display a composite image obtained by combining the camera 3D image acquired from the medical imaging device and the space CG for making the user feel more spatial reproduction.

The space CG is an image different from the camera 3D image, and includes a base unit for making the user feel a sense of depth, a timer and intraoperative information such as a vital sign, a reference information part indicating reference information such as preoperative information such as an X-ray image and an MRI image stored in advance, and an interactive part that can be interactively disposed and rotated, such as a blood vessel CG. The base unit is preferably a space CG configured to be felt by the user such that there is a vanishing point in the depth direction of the screen.

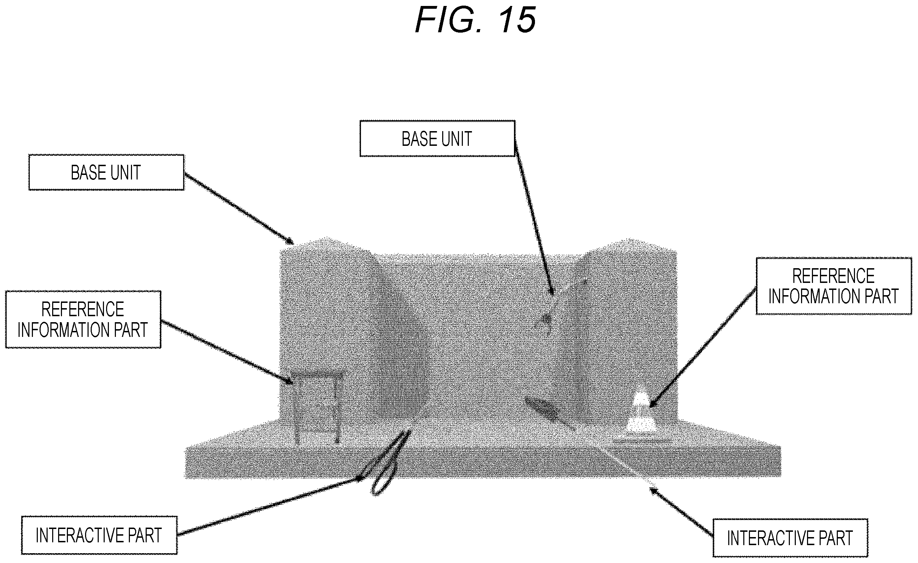

Fig. 15 is a diagram illustrating an example of the space CG. The space CG includes a base unit, a reference information part, and an interactive part. The base unit is a part that does not change even when the camera 3D image changes. The reference information part is a part that displays reference information such as a vital sign. The interactive part is a part that can be interactively displayed on the basis of user's control or the like.

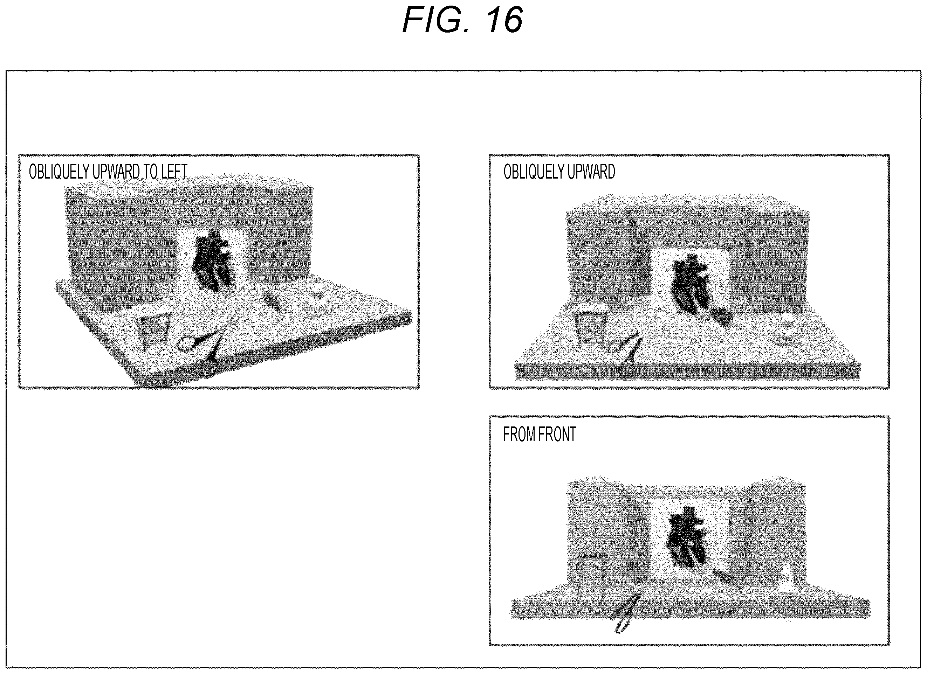

Fig. 16 is a diagram illustrating a difference in the composite image depending on the viewpoint position. Fig. 16 is a diagram assuming a case where a composite image is virtually captured obliquely upward to the left, obliquely upward, or from the front when the user directly faces the display device.

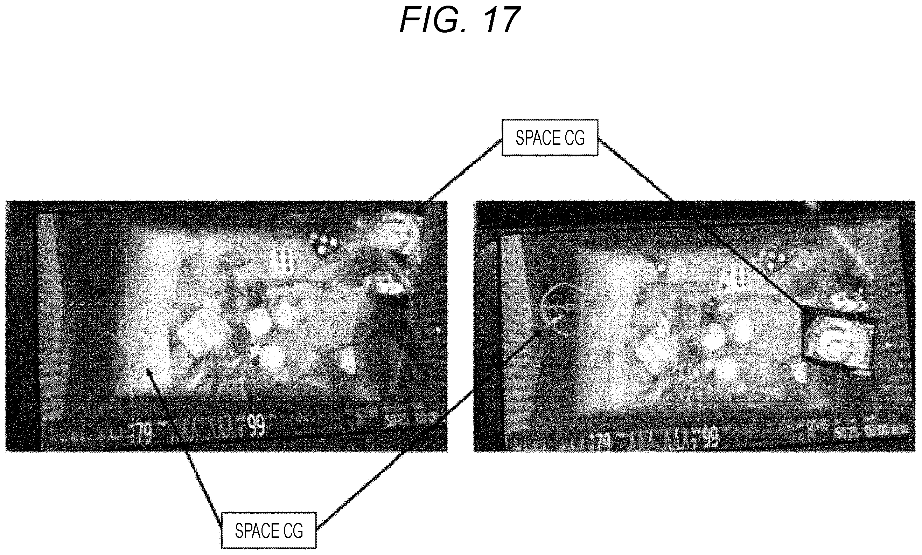

Fig. 17 is a diagram illustrating an example of a composite image. A white polygonal line that the user feels as if there is a right angle in the front direction of the screen is displayed so that the user feels the stereoscopic effect more. The polygonal line may be a color other than white. The polygonal line is displayed such that the bent portion follows the movement of the user's eyes and face. As a result, the user can recognize as if an object exists in a more front direction of the screen on the spatial reproduction display. Two polygonal lines are preferably displayed. When two polygonal lines are displayed, it is preferable to display the surgical video between the two polygonal lines and to display the reference information such as the X-ray image in a region other than between the two polygonal lines.

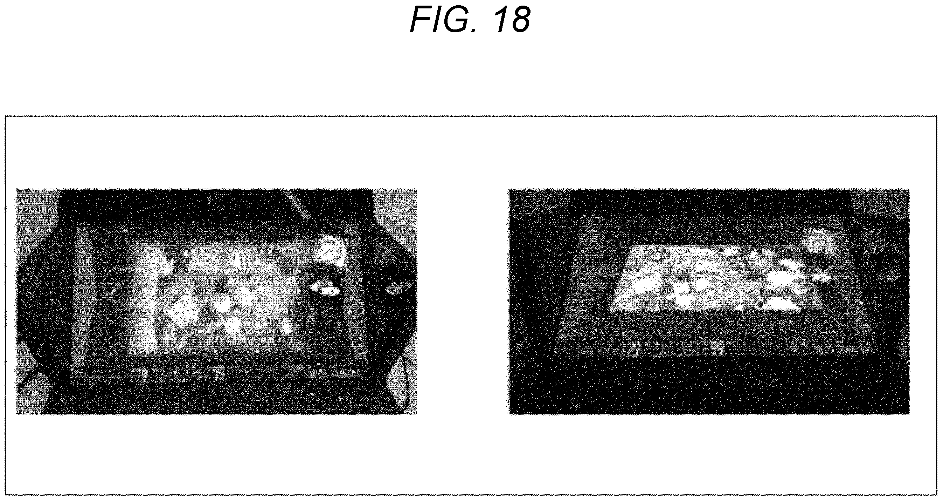

Fig. 18 is a diagram illustrating an example of a composite image. The left drawing in Fig. 18 is an example in which a camera image is displayed to face a viewpoint position of the user, and the right drawing in Fig. 18 is an example in which the 3D image is displayed to be fixed regardless of the viewpoint position of the user. The camera 3D image and the space CG may be displayed so as to always follow the viewpoint position and the face position of the user and face the user, or any image may be fixedly displayed.

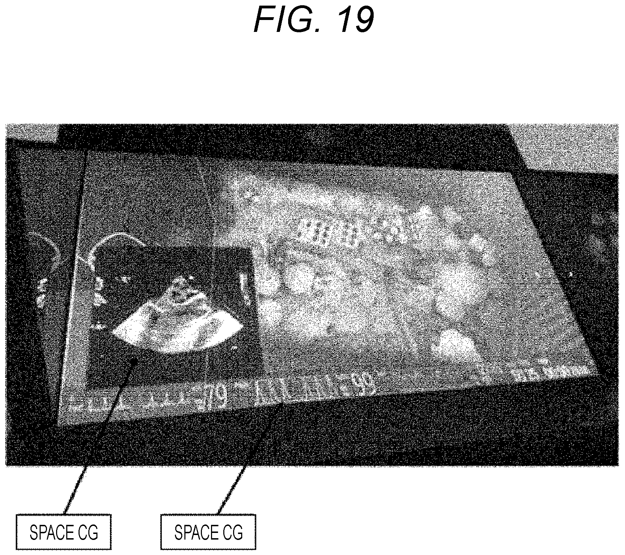

Fig. 19 is a diagram illustrating an example of a composite image. As illustrated in Fig. 19, the space CG may be superimposed and displayed so that the user feels that the space CG is disposed in the front direction of the screen with respect to the camera 3D image. As a result, since the planar image is displayed on the front side and the stereoscopic image is displayed on the back side, the user can feel a more stereoscopic effect. In particular, it is preferable that the preoperative information and the intraoperative information are displayed on the front side. As a result, the user can perform the surgery while always putting the preoperative information and the intraoperative information in the field of view.

Note that the reference information part including the intraoperative information and the timer displayed at the lower part of the screen may be displayed in a dark color only when the user's viewpoint overlaps the reference information part, and may be displayed in a translucent color in other cases.

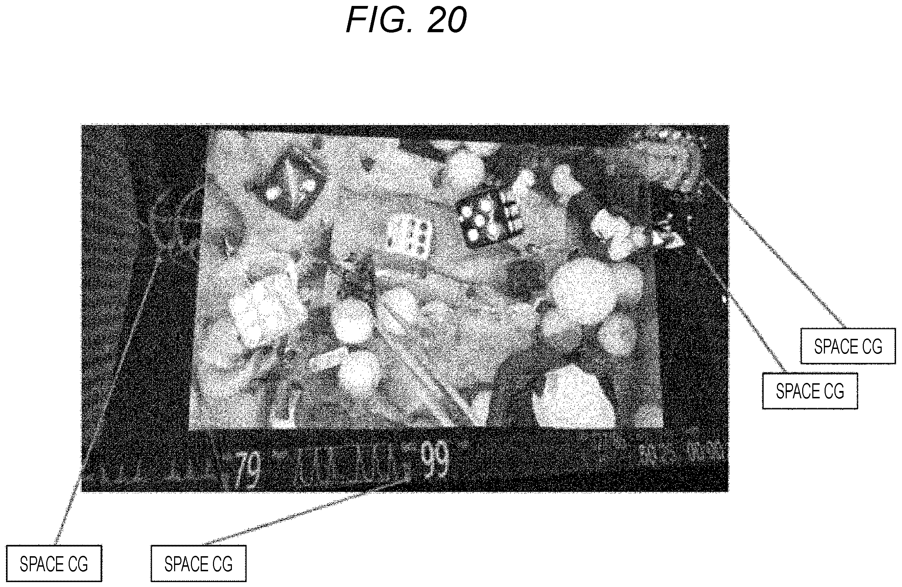

Fig. 20 is a diagram illustrating an example of a composite image. As illustrated in Fig. 20, it is preferable that a surgical video (camera 3D image), preoperative information, intraoperative information (reference information part), and a CG model (interactive part) are virtually disposed and displayed on a spatial video (base unit) as the composite image. The CG model is, for example, a 3D model generated from preoperative information.

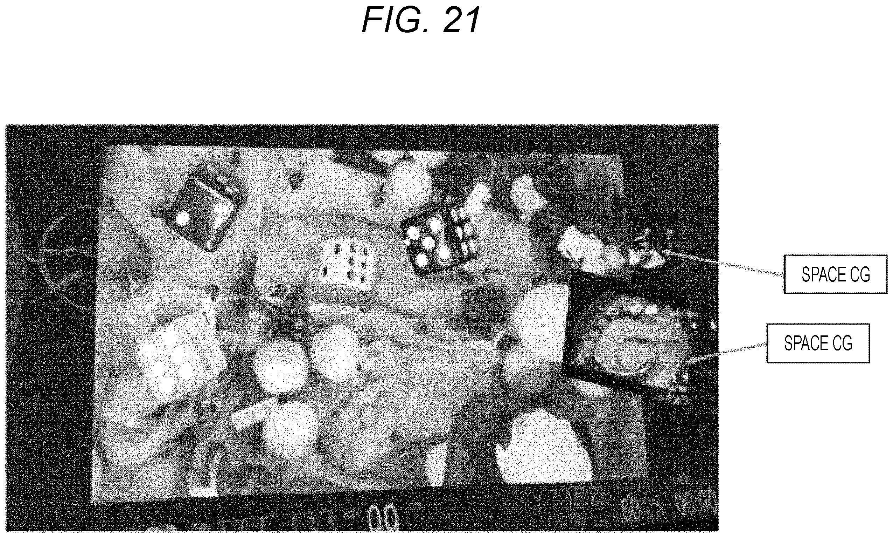

Fig. 21 is a diagram illustrating an example of a composite image. As illustrated in Fig. 21, the composite image may have a layer structure. For example, when a plurality of X-ray images is displayed as preoperative information which is a reference information part, it is preferable that the second X-ray image is virtually spatially arranged on the front side of the first X-ray image. The old image may be displayed on the back side of the surgical video. As a result, it is possible to prevent the surgical video from being invisible due to the reference information part already confirmed by the user.

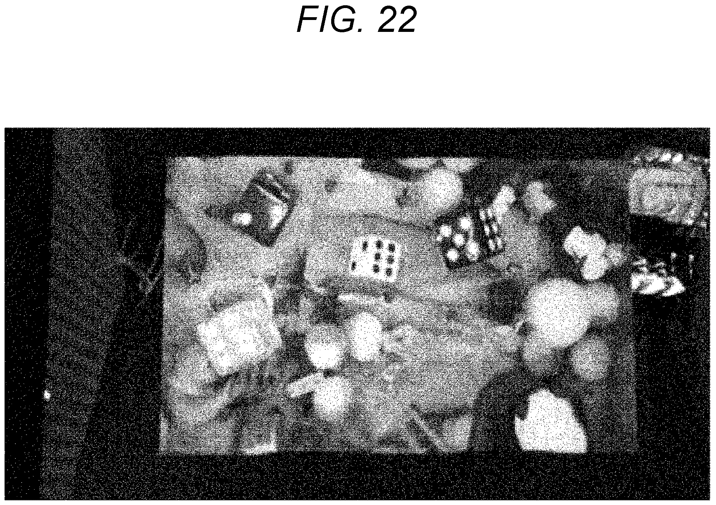

Fig. 22 is a diagram illustrating an example of a composite image. As illustrated in Fig. 22, the display of the composite image may be changed on the basis of the priority for each layer on the basis of an angle at which the user views the screen or the line-of-sight. For example, it is preferable that the surgical video is a base layer having a high priority of being visible at any angle or line-of-sight position.

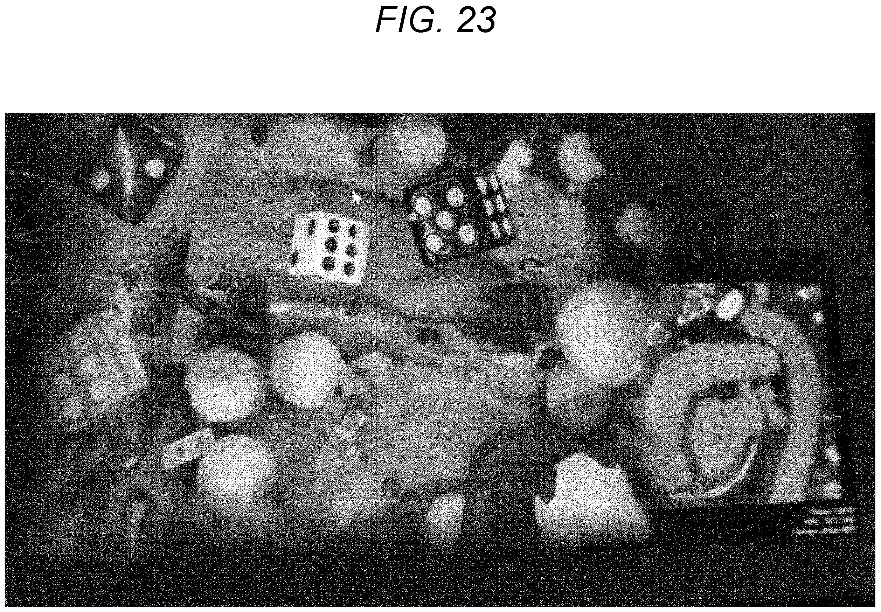

Fig. 23 is a diagram illustrating an example of a composite image. As illustrated in Fig. 23, the composite image may be switched from the 3D display to the 2D display in a case where the position of the face deviates from a predetermined range. As a result, the 3D defect caused by the deviation of the position of the face from the predetermined range can be avoided, and the user can always view the composite image.

In a case where the position of the face deviates from the predetermined range, the method of displaying the preoperative information may be changed. For example, it is preferable to increase the transmittance of the preoperative information that has been displayed translucently as the position of the face deviates from the predetermined range. As a result, it is possible to avoid a situation in which it is difficult for the user to visually recognize the surgical video because the preoperative information overlaps the surgical video.

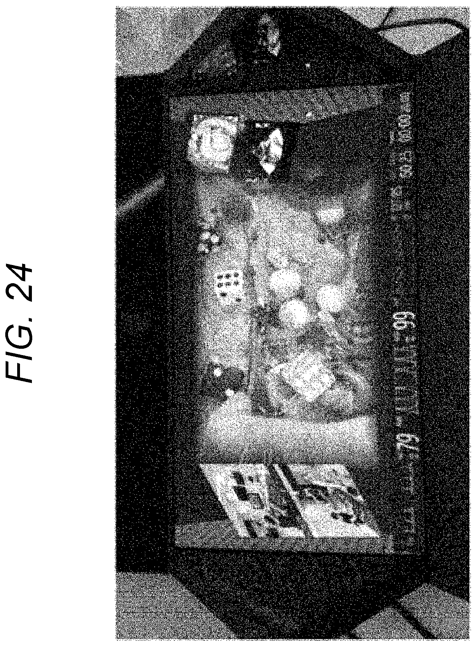

Fig. 24 is a diagram illustrating an example of a composite image. As illustrated in Fig. 24, in the composite image, surgery room information including a state of a surgery room may be displayed as a reference information part.

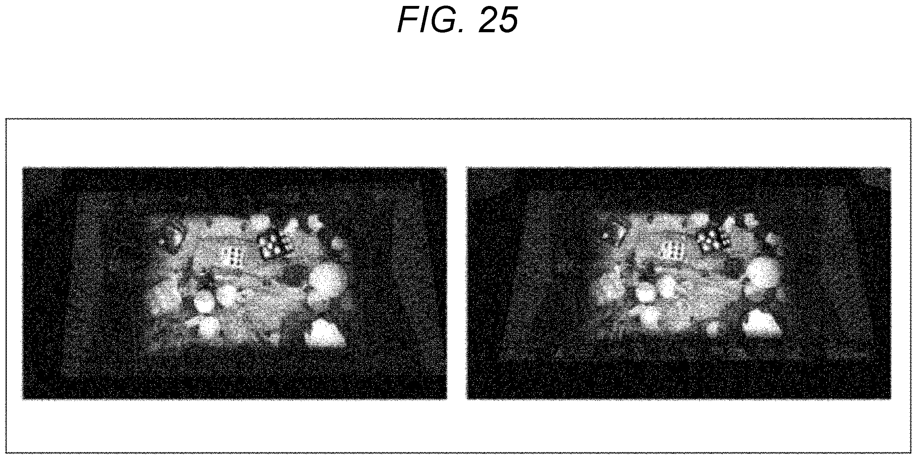

Fig. 25 is a diagram illustrating an example of a composite image. The left drawing in Fig. 25 is a diagram illustrating a case where the angle (parallax) of the composite image is decreased, and the right drawing in Fig. 25 is a diagram illustrating a case where the angle of the composite image is increased. When the angle of the composite image is increased, the user can feel the depth direction more.

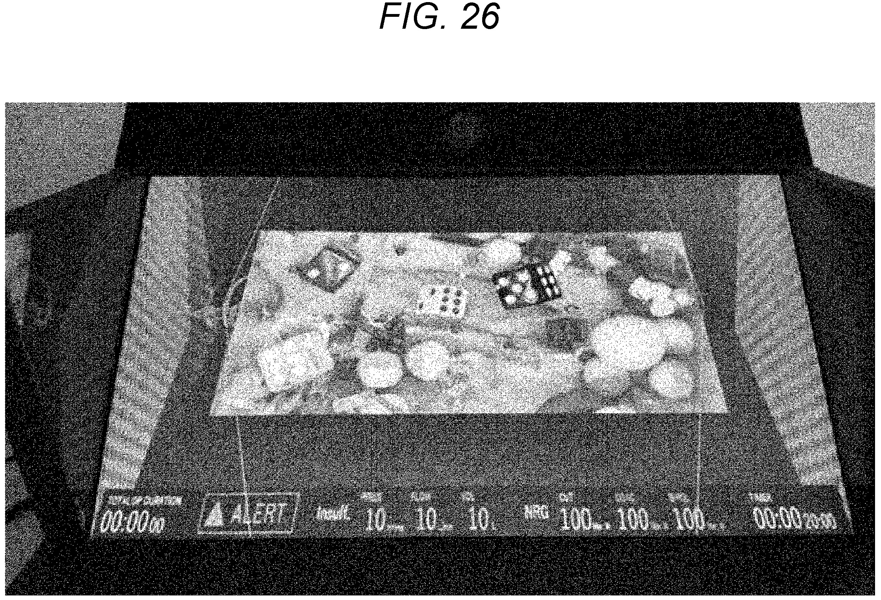

Fig. 26 is a diagram illustrating an example of a composite image. As illustrated in Fig. 26, it is preferable that the virtual display surface of the surgical video (base layer) of the composite image (stereoscopic video) is displayed so as to be parallel to an angle of looking into the display surface of the user.

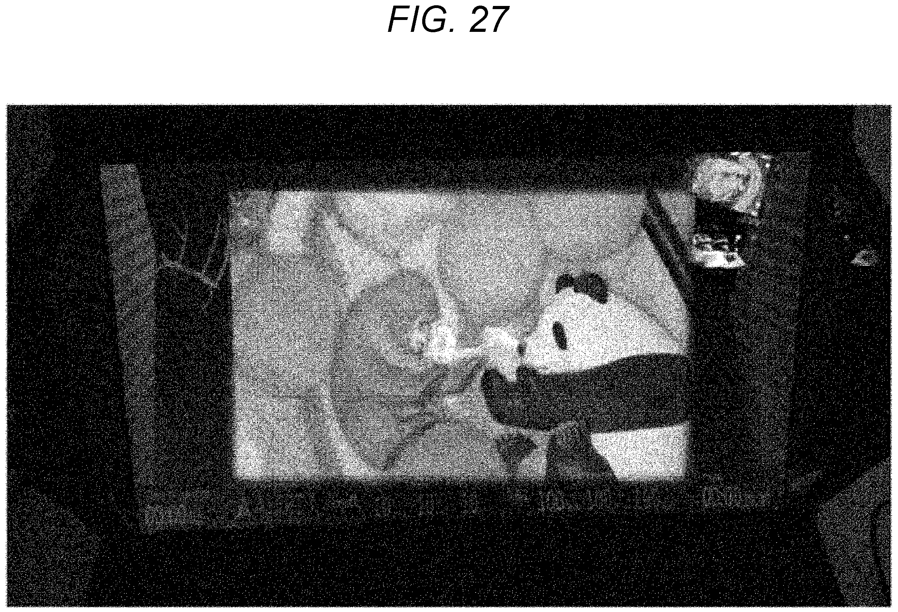

Fig. 27 is a diagram illustrating an example of a composite image. As illustrated in Fig. 27, in a case where a CG image is superimposed on a surgical video, it is preferable that the CG image is disposed at a predetermined position of the surgical video while being moved. As a result, the user can easily grasp which position of the surgical video the CG image matches and is superimposed on in the depth direction.

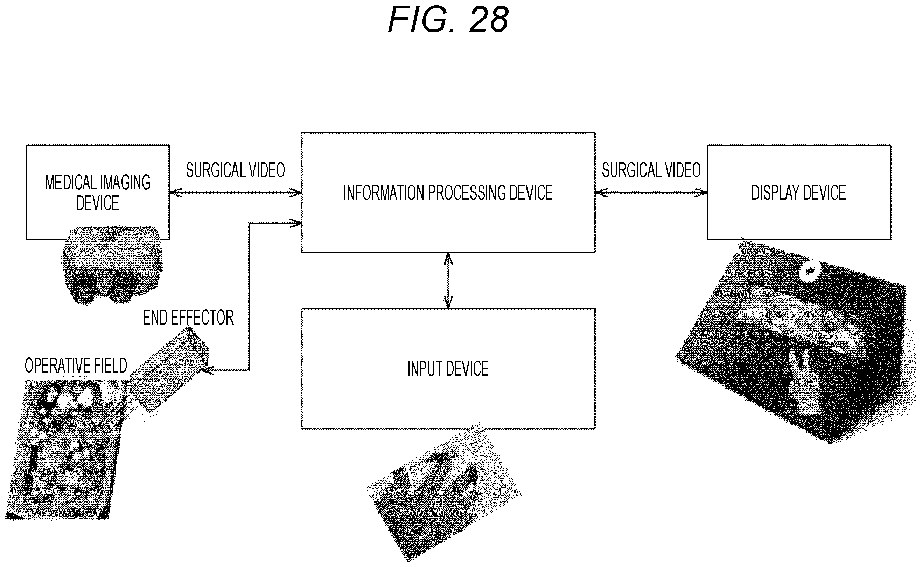

Fig. 28 is a diagram illustrating another configuration example of the surgical robot system that displays a CG image on a composite image in conjunction with the input device. As illustrated in Fig. 28, the surgical robot system of the present embodiment can provide the sense that the user directly interferes with the object on the more virtual space by indicating the movement of the hand or the movement of the finger of the user on the display device in conjunction with the output of the input device capable of detecting the movement of the hand or the movement of the finger of the user.

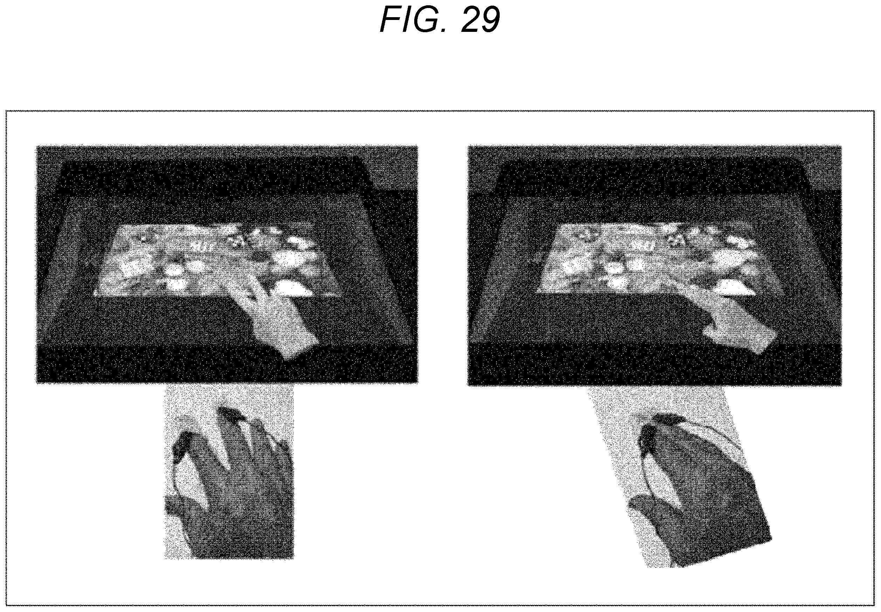

At this time, a CG indicating the movement of the hand or the movement of the finger of the user may be displayed in a superimposed manner with respect to the region where the end effector is shown. For example, as illustrated in Fig. 29, it is preferable to configure such that the hand eye coordination matches on the screen.

Voice recognition or line-of-sight recognition may be added as the input device.

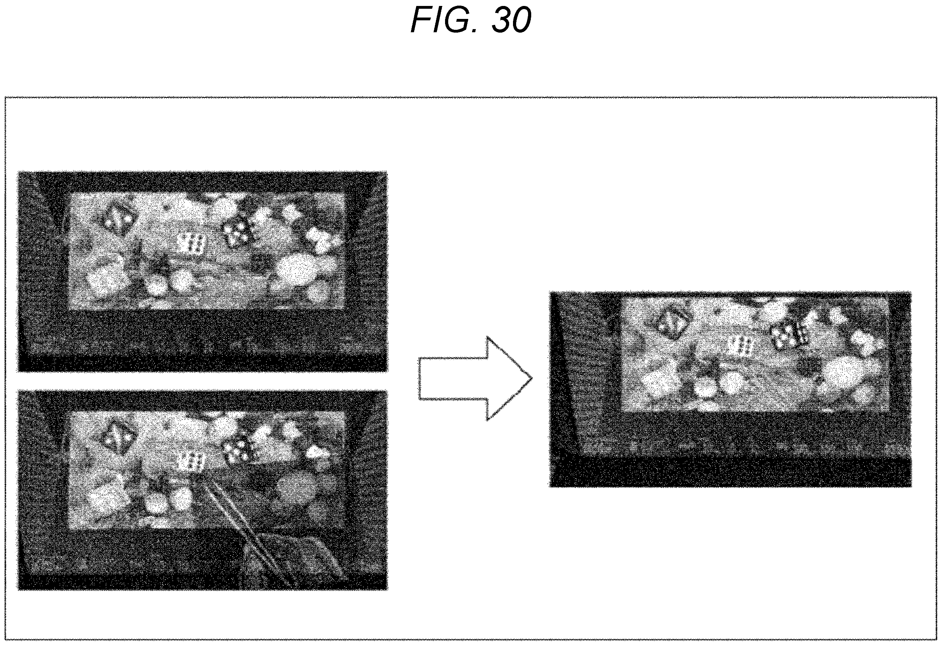

Fig. 30 is a diagram illustrating an example of a composite image. As illustrated in Fig. 30, a CG image may be combined with the composite image. For example, a CG image indicating the motion of the hand or the motion of the finger may be superimposed on the screen, and the CG image may be changed in conjunction with fixation or user input.

In the present specification, the system represents the entire device including a plurality of devices.

Note that the effects described in the present specification are merely examples and are not limited, and there may be other effects.

Note that the embodiments of the present technology are not limited to the above-described embodiments, and various changes can be made without departing from the gist of the present technology.

Note that the present technology can also have the following configuration.

(1) A surgical robot system including:

a spatial reproduction display capable of displaying a surgical video visually recognized as a stereoscopic image in a stereoscopic space formed by a plane through which a lower side of a display surface of a monitor and a normal surface of an installation surface pass and a plane through which an upper side of the display surface and a parallel surface of the installation surface pass;
an imaging device that captures the surgical video;
an operating device connected to the spatial reproduction display; and
a surgical robot that operates by an operation of an operator via the operating device.

(2) The surgical robot system according to (1) described above, in which
the spatial reproduction display displays a composite image obtained by combining the surgical video, reference information indicating preoperative information or intraoperative information, and a base unit that increases stereoscopic effect of the surgical video.

(3) The surgical robot system according to (1) or (2) described above, in which
the spatial reproduction display displays a composite image obtained by combining the surgical video, reference information indicating preoperative information or intraoperative information, a base unit that increases stereoscopic effect of the surgical video, and an interactive part that is interactively placeable, movable or rotatable on the basis of an operation of a user.

(4) The surgical robot system according to any one of (1) to (3) described above, in which
the spatial reproduction display displays a composite image arranged on a virtual space such that reference information indicating preoperative information or intraoperative information is displayed in front of the surgical video.

(5) The surgical robot system according to (1) to (4) described above, in which
the spatial reproduction display displays a composite image arranged on a virtual space such that reference information indicating preoperative information or intraoperative information is displayed on a back side of the surgical video.

REFERENCE SIGNS LIST

| | |
|---|---|
| 1 | Information processing device |
| 10 | Display unit |
| 12 | Display surface |
| 20 | Display surface detection unit |
| 30 | User detection unit |
| 40 | Storage unit |
| 50 | Control unit |
| 52 | Display surface information acquisition unit |
| 54 | User information acquisition unit |
| 56 | Content acquisition unit |
| 58 | Display control unit |
| 92 | Display surface |
| 100 | Master device |
| 101 | Information processing device |
| 122 | Lower end |
| 124 | Upper end |
| 200 | Input device |
| 300 | Support base |
| 400 | Display device |
| 500 | Slave device |
| 501 | Information processing device |
| 600 | Medical device |
| 700 | Arm |

**Claims**

1. A surgical robot system comprising:

a spatial reproduction display capable of displaying a surgical video visually recognized as a stereoscopic image in a stereoscopic space formed by a plane through which a lower side of a display surface of a monitor and a normal surface of an installation surface pass and a plane through which an upper side of the display surface and a parallel surface of the installation surface pass;
an imaging device that captures the surgical video;
an operating device connected to the spatial reproduction display; and
a surgical robot that operates by an operation of an operator via the operating device.

2. The surgical robot system according to claim 1, wherein
the spatial reproduction display displays a composite image obtained by combining the surgical video, reference information indicating preoperative information or intraoperative information, and a base unit that increases stereoscopic effect of the surgical video.

3. The surgical robot system according to claim 1, wherein
the spatial reproduction display displays a composite image obtained by combining the surgical video, reference information indicating preoperative information or intraoperative information, a base unit that increases stereoscopic effect of the surgical video, and an interactive part that is interactively placeable, movable or rotatable on a basis of an operation of a user.

4. The surgical robot system according to claim 1, wherein
the spatial reproduction display displays a composite image arranged on a virtual space such that reference information indicating preoperative information or intraoperative information is displayed in front of the surgical video.

5. The surgical robot system according to claim 1, wherein
the spatial reproduction display displays a composite image arranged on a virtual space such that reference information indicating preoperative information or intraoperative information is displayed on a back side of the surgical video.

EP 4 467 095 A1

# FIG. 1

# FIG. 2

# FIG. 3

EP 4 467 095 A1

*FIG. 4*

## FIG. 5

# FIG. 6

INFORMATION PROCESSING DEVICE

1

20
DISPLAY SURFACE
DETECTION UNIT

30
USER
DETECTION UNIT

50
CONTROL UNIT

52
DISPLAY SURFACE
INFORMATION
ACQUISITION UNIT

54
USER INFORMATION
ACQUISITION UNIT

56
CONTENT
ACQUISITION UNIT

58
DISPLAY
CONTROL UNIT

40
STORAGE UNIT

10
DISPLAY UNIT

12
DISPLAY SURFACE

# FIG. 7

# FIG. 8

# FIG. 9

```
                    ┌─────────────┐
                    │    START    │
                    └──────┬──────┘
                           ▼
┌──────────────────────────────────────────────┐ S102
│      ACQUIRE DISPLAY SURFACE INFORMATION       │
└───────────────────────┬────────────────────────┘
                        ▼
┌──────────────────────────────────────────────┐ S104
│               SET DRAWING SPACE                │
└───────────────────────┬────────────────────────┘
                        ▼
┌──────────────────────────────────────────────┐ S106
│  DISPOSE FLOOR SURFACE, WALL SURFACE, AND OBJECT │
└───────────────────────┬────────────────────────┘
                        ▼
┌──────────────────────────────────────────────┐ S108
│            ACQUIRE USER INFORMATION            │
└───────────────────────┬────────────────────────┘
                        ▼
┌──────────────────────────────────────────────┐ S110
│      PERFORM RENDERING WITH VIRTUAL VIEWPOINT    │
└───────────────────────┬────────────────────────┘
                        ▼
┌──────────────────────────────────────────────┐ S112
│     GENERATE IMAGE ON VIRTUAL DISPLAY SURFACE    │
└───────────────────────┬────────────────────────┘
                        ▼
┌──────────────────────────────────────────────┐ S114
│ ACQUIRE DISPLAY IMAGE WITH ORTHOGRAPHIC PROJECTION │
└───────────────────────┬────────────────────────┘
                        ▼
┌──────────────────────────────────────────────┐ S116
│                   DISPLAY                      │
└───────────────────────┬────────────────────────┘
                        └──────────────►
```

# FIG. 10

EP 4 467 095 A1

# FIG. 11

# FIG. 12

MEDICAL IMAGING DEVICE —SURGICAL VIDEO→ INFORMATION PROCESSING DEVICE ←SURGICAL VIDEO→ DISPLAY DEVICE

END EFFECTOR

OPERATIVE FIELD

INPUT DEVICE

EP 4 467 095 A1

# FIG. 13

CAMERA 3D IMAGE

SPATIAL REPRODUCTION CG IMAGE
FROM VIEWPOINT POSITION

HYBRID COMPOSITE IMAGE (3D SPACE)

# FIG. 14

CAMERA 3D IMAGE

REFERENCE INFORMATION PART
SPACE CG

REFERENCE INFORMATION PART
SPACE CG

INTERACTIVE PART
SPACE CG

BASE UNIT
SPACE CG

REFERENCE INFORMATION PART
SPACE CG

EP 4 467 095 A1

FIG. 15

# FIG. 16

OBLIQUELY UPWARD TO LEFT

OBLIQUELY UPWARD

FROM FRONT

## FIG. 17

SPACE CG

SPACE CG

FIG. 18

# FIG. 19

*FIG. 20*

SPACE CG

SPACE CG

SPACE CG

SPACE CG

## FIG. 21

SPACE CG

SPACE CG

FIG. 22

*FIG. 23*

*FIG. 24*

*FIG. 25*

## FIG. 26

EP 4 467 095 A1

*FIG. 27*

## FIG. 28

| MEDICAL IMAGING DEVICE | → SURGICAL VIDEO → | INFORMATION PROCESSING DEVICE | → SURGICAL VIDEO → | DISPLAY DEVICE |

OPERATIVE FIELD

END EFFECTOR

INPUT DEVICE

FIG. 29

EP 4 467 095 A1

*FIG. 30*

# INTERNATIONAL SEARCH REPORT

| International application No. |
|---|
| **PCT/JP2023/000118** |

**A. CLASSIFICATION OF SUBJECT MATTER**

*A61B 34/10*(2016.01)i; *A61B 34/20*(2016.01)i; *A61B 34/37*(2016.01)i; *H04N 13/302*(2018.01)i; *H04N 13/361*(2018.01)i
FI: A61B34/20; H04N13/302; H04N13/361; A61B34/37; A61B34/10

According to International Patent Classification (IPC) or to both national classification and IPC

**B. FIELDS SEARCHED**

Minimum documentation searched (classification system followed by classification symbols)

A61B34/10; A61B34/20; A61B34/37; H04N13/302; H04N13/361

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 2020-162916 A (SONY CORP.) 08 October 2020 (2020-10-08) | 1 |
| | paragraphs [0002], [0003], [0019]-[0021], [0026]-[0031], [0039]-[0041], fig. 1-3, 7 | |
| Y | | 2-5 |
| Y | JP 2015-19679 A (SEIKO EPSON CORP.) 02 February 2015 (2015-02-02) | 2-5 |
| | paragraphs [0084], [0120], fig. 14, 18 | |
| A | | 1 |
| Y | JP 2021-177580 A (SONY GROUP CORP.) 11 November 2021 (2021-11-11) | 2-3 |
| | paragraphs [0017], [0018], fig. 1, 2 | |
| A | | 1, 4-5 |
| Y | JP 2010-525838 A (NEUROARM SURGICAL LTD.) 29 July 2010 (2010-07-29) | 3 |
| | paragraphs [0044], [0051], [0055] | |
| A | | 1-2, 4-5 |

☑ Further documents are listed in the continuation of Box C.  ☑ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **07 February 2023** | **21 February 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**

International application No.

**PCT/JP2023/000118**

| C. | DOCUMENTS CONSIDERED TO BE RELEVANT | |
|---|---|---|
| Category* | Citation of document, with indication, where appropriate, of the relevant passages | Relevant to claim No. |
| Y | JP 2019-531117 A (VERB SURGICAL INC.) 31 October 2019 (2019-10-31) paragraph [0084], fig. 18 | 3 |
| A | | 1-2, 4-5 |

Form PCT/ISA/210 (second sheet) (January 2015)

# EP 4 467 095 A1

International application No.

**PCT/JP2023/000118**

| Patent document cited in search report | | | Publication date (day/month/year) | Patent family member(s) | Publication date (day/month/year) |
|---|---|---|---|---|---|
| JP | 2020-162916 | A | 08 October 2020 | US 2022/0060678 A1 paragraphs [0002], [0003], [0043]-[0046], [0050]-[0056], [0066]-[0068], fig. 1-3, 7 CN 113613847 A | |
| JP | 2015-19679 | A | 02 February 2015 | US 2016/0154620 A1 paragraph [0153], [0209], fig. 14, 18 CN 104298344 A KR 10-2016-0033721 A | |
| JP | 2021-177580 | A | 11 November 2021 | (Family: none) | |
| JP | 2010-525838 | A | 29 July 2010 | US 2010/0198402 A1 paragraphs [0051], [0057], [0059] | |
| JP | 2019-531117 | A | 31 October 2019 | US 2018/0092706 A1 paragraph [0105], fig. 18 CN 108289600 A KR 10-2019-0043143 A | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2019097890 A